# EUROPEAN PATENT APPLICATION

(11) **EP 0 958 805 A2**
(43) Date of publication of application: **24.11.1999**
(21) Application number: 99401080.9
(22) Date of filing: 03.05.1999
(51) Int. Cl.: A61K 7/02, A61K 7/48

(54) **Makeup cosmetic composition**

(30) Priority: 20.05.1998 JP 15536498
(71) Applicant: SHIN-ETSU CHEMICAL CO., LTD., Chiyoda-ku Tokyo (JP)
(72) Inventor: Kuwata, Satoshi, c/o Shin-Etsu Chemical Co., Ltd., Usui-gun, Gunma-ken (JP); Inokuchi, Yoshinori, c/o Shin-Etsu Chem. Co., Ltd., Usui-gun, Gunma-ken (JP)
(74) Representative: Armengaud Ainé, Alain

(57) **Abstract**

Disclosed is a novel makeup cosmetic composition which can be in a variety of preparation forms such as foundations and cheek rouges capable of imparting users thereof with an excellent feeling of use not only during but also after the cosmetic makeup finishing. Characteristically, the makeup cosmetic composition of the invention is compounded, besides a non-silicone powder ingredient and an oiling agent, with a limited amount of a powder of composite silicone particles, each particle consisting of a core of a cured silicone rubber and a cladding layer of a polyorganosilsesquioxane. The composite silicone particles can be prepared by the hydrolysis-condensation reaction of an organotrialkoxy silane in an aqueous suspension of particles of a cured silicone rubber prepared by the hydrosilation reaction between a vinyl-containing organopolysiloxane and an organohydrogen polysiloxane as jointly emulsified in an aqueous medium.

## Description

### BACKGROUND OF THE INVENTION

The present invention relates to a novel makeup cosmetic composition or, more particularly, to a powder-containing makeup cosmetic composition having good spreadability over the human skin and capable of imparting the users with excellent feeling of use in respect of non-stickiness and refreshingness.

It is known in the prior art that certain makeup cosmetic compositions are formulated with fine particles of a silicone such as fine particles of a polyorganosilsesquioxane resin and fine particles of a cured silicone rubber having elasticity (see, for example, Japanese Patent Kokai 1-268615, Japanese Patent Publication 7-053646 and elsewhere).

Since fine particles of the above mentioned polyorganosilsesquioxane resin have a high hardness, users of a makeup cosmetic composition containing such particles are usually imparted with a hard and disordered feeling of use though being free from a feeling of use with stickiness. The fine particles of a cured silicone rubber are, though free from the above mentioned disadvantages inherent in the polyorganosilsesquioxane resin particles, disadvantageous in respect of the workability in the preparation of a cosmetic composition because of their poor flowability to cause difficulties in handling and strong agglomeration of particles resulting in low dispersibility in various base ingredients of the cosmetic composition along with poor compatibility thereof with the base ingredients. In this regard, the inventors previously conducted studies on the use of a powder of composite silicone particles (see Japanese Patent Kokai 9-20631).

### SUMMARY OF THE INVENTION

In view of the above described problems in the conventional makeup cosmetic compositions formulated with a silicone powder in general, the present invention accordingly has an object to provide a novel and improved makeup cosmetic composition containing a silicone powder having good affinity to the other ingredients in the makeup cosmetic composition so as to be capable of exhibiting good spreadability over the human skin and imparting the users thereof with a pleasant and comfortable feeling of use with non-stickiness and refreshingness. In this regard, the inventors previously conducted studies on the application of a powder of composite silicone particles (see Japanese Patent Kokai 9-20631).

Thus, the makeup cosmetic composition of the present invention comprises, as a uniform blend:
(A) from 0.01 to 30% by weight of a powder of composite silicone particles, each particle consisting of a core of a cured silicone rubber and a cladding layer of a polyorganosilsesquioxane resin formed on the core;
(B) a non-silicone powder; and
(C) an oiling agent.

### DETAILED DESCRIPTION OF THE PREFERRED EMBODIMENTS

As is described above, the essential ingredients in the inventive makeup cosmetic composition include the above described components (A), (B) and (C), of which the most characteristic ingredient is the component (A) which is a powder of composite silicone particles, each particle consisting of a core of a cured silicone rubber and a cladding layer of a polyorganosilsesquioxane resin formed on the core.

The composite silicone particles as the component (A) can be prepared by a process described below.

In the first place, an oil-in-water aqueous emulsion is prepared by emulsifying a vinyl group-containing organopolysiloxane and an organohydrogenpolysiloxane in combination in an aqueous medium containing an emulsifying agent with admixture of a catalytic amount of a platinum compound as a catalyst for the hydrosilation reaction to form spherical fine particles of a cured silicone rubber as dispersed in the aqueous medium. The spherical particles of the cured silicone rubber obtained in this way should have a particle diameter in the range from 0.1 to 100 µm when to be used in the present invention. In the next place, the aqueous suspension of the cured silicone rubber particles is admixed with an organotrialkoxy silane compound such as methyl trimethoxy silane and an alkaline compound as a catalyst to effect hydrolysis-condensation reaction of the organotrialkoxysilane compound depositing a cladding layer of a polyorganosilsesquioxane resin on the particles of the cured silicone rubber as the core followed by separation of the particles from the aqueous medium and drying thereof to give fine spherical particles suitable as the component (A), each particle consisting of a core of the cured silicone rubber and a cladding layer of the polyorganosilsesquioxane resin on the core. This process is known in the art and described, for example, in Japanese Patent Kokai 7-196815.

The amount of the composite silicone particles formulated in the inventive makeup cosmetic composition is in the range from 0.01 to 30% by weight or, preferably, from 0.05 to 10% by weight. When the amount thereof is too small, the desired improvements in the makeup cosmetic composition cannot be fully obtained as a matter of course while, when the amount thereof is too large, the makeup cosmetic composition would be inferior in respect of use feasibility.

The component (B) is a non-silicone powder material which can be selected from a great variety of powder materials formulated in conventional makeup cosmetic compositions including inorganic and organic powders, of which the particle diameter, particle configuration, which may be spherical, needle-formed and platelet-formed, and particle structure, which may be porous or non-porous, are not particularly limitative and can be selected depending on the particular types of the cosmetic compositions.

Examples of the inorganic powder materials include titanium dioxide, zirconium oxide, zinc oxide, cerium oxide, magnesium oxide, barium sulfate, calcium sulfate, magnesium sulfate, calcium carbonate, magnesium carbonate, talc, natural and synthetic micas, kaolin sericite, muscovite, phlogopite, lepidolite, biotite, lithia mica, silicic acid, anhydrous silicic acid, aluminum silicate, magnesium silicate, aluminum magnesium silicate, calcium silicate, barium silicate, strontium silicate, metal tungstates, hydroxyapatite, vermiculite, bentonite, montmorillonite, pectolite, zeolite, ceramic powders, calcium monohydrogenphosphate, alumina, aluminum hydroxide, boron nitride and silica.

Examples of organic powders include, as a first class, powders of synthetic and natural polymers such as polyamides, polyesters, polyethylenes, polypropylenes, polystyrenes, polyurethanes, benzoguanamine resins, polymethyl benzoguanamine resins, polytetrafluoroethylenes, polymethyl methacrylates, cellulose, silk, nylon resins, i.e. 12-nylon and 6-nylon, styrene-acrylic acid copolymeric resins, styrene-divinylbenzene copolymeric resins, vinyl resins, urea resins, phenol resins, fluorocarbon resins, acrylic resins, melamine resins, epoxy resins, polycarbonate resins, microcrystalline cellulose, rice starch and lauroyl lysine.

Examples of the organic powders also include powders of a metal salt of a surface active agent or so-called metal soaps such as zinc stearate, aluminum stearate, calcium stearate, magnesium stearate, zinc myristate, magnesium myristate, zinc cetylphosphate, calcium cetylphosphate and sodium zinc cetylphosphate.

The powder material as the component (B) can be a pigment including inorganic colored pigments such as red iron oxide, iron hydroxide, iron titanate, γ-iron oxide, yellow iron oxide, ocher, black iron oxide, carbon black, mango violet, cobalt violet, chromium hydroxide, chromium oxide, cobalt oxide, cobalt titanate, Prussian blue and ultramarine and organic lake pigments obtained by lake-formation of a tar dye or natural dye as well as composite pigments thereof.

Suitable powder materials as the component (B) further include pearlescent pigments such as titanium dioxide-coated mica flakes, bismuth oxychloride, titanium dioxide-coated bismuth oxychloride, titanium dioxide-coated talc, fish scale flakes and titanium dioxide-coated colored mica flakes, powders of a metal such as aluminum, copper and stainless steel and synthetic and natural dyes such as Red #3. Red #104, Red #106, Red #201, Red #202. Red #204, Red #205, Red #220, Red #226, Red #227, Red #228, Red #230, Red #401, Red #505, Yellow #4, Yellow #5, Yellow #202, Yellow #203, Yellow #204, Yellow #401, Blue #1, Blue #2, Blue #201, Blue #404, Green #3, Green #201, Green #204, Green #205, Orange #201, Orange #203, Orange #204, Orange #206, Orange #207, carminic acid, laccaic acid, carthamin, brazilin and crocin.

The above named various powder materials can be used as the component (B) either singly or as a combination of two kinds or more according to need. Further, the powder materials can be compounded in the cosmetic composition as such, in a composite form of two kinds or more or after a surface treatment with an oiling agent or a silicone or fluorocarbon compound.

The component (C) in the inventive makeup cosmetic composition is an oiling agent which can be selected from a variety of oily materials conventionally compounded in makeup cosmetic compositions without particular limitations.

Suitable oily materials include solid, semi-solid and liquid ones originating in plants and animals as well as mineral oils and synthetic oils. Examples of suitable natural oily materials and semi-synthetic oily materials derived therefrom as an oiling agent include avocado oil, linseed oil, almond oil, insect wax, perilla oil, olive oil, cacao butter, kapok wax, *kaya* oil, carnauba wax, cod liver oil, candelilla wax, beef tallow, hydrogenated beef tallow, apricot kernel oil, spermaceti, hydrogenated oil, wheat germ oil, sesame oil, rice germ oil, rice bran oil, sugar cane wax, *sasanqua* oil, safflower oil, shear butter, Chinese tung oil, cinnamon oil, *jojoba* wax, shellac wax, turtle oil, soybean oil, tea seed oil, *tsubaki* oil, evening primrose oil, corn oil, lard, rape seed oil, Japanese tung oil, bran wax, germ oil, horse oil, persic oil, palm oil, palm kernel oil, castor oil, hydrogenated castor oil, castor oil fatty acid methyl ester, sunflower seed oil, grape seed oil, bayberry wax, *jojoba* oil, macadamia nut oil, beeswax, mink oil, cottonseed oil, cotton wax, japan wax, montan wax, coconut oil, hydrogenated coconut oil, tri(coconut oil fatty acid) glyceride, mutton tallow, peanut oil, lanolin, liquid lanolin, reduced lanolin, lanolin alcohol, hard lanolin, lanolin acetate, lanolin fatty acid isopropyl ester, hexyl laurate, POE lanolin alcohol ethers, POE lanolin alcohol acetate, lanolin fatty acid polyethyleneglycol esters, POE hydrogenated lanolin alcohol ethers and egg yolk oil.

Hydrocarbon oils suitable as the component (C) include ozokerite, squalane, squalene, ceresin, paraffin, paraffin wax, liquid paraffin, pristane, polyisobutylene, microcrystalline wax and Vaseline. Higher fatty acids suitable as the component (C) include lauric acid, myristic acid, palmitic acid, stearic acid, behenic acid, undecylenic acid, oleic acid, linoleic acid, linolenic acid, arachidonic acid, eicosapentaenoic acid, docosahexanoenic acid, isostearic acid and 12-hydroxystearic acid. Higher alcohols suitable as the component (C) include lauryl al-cohol, myristyl alcohol, palmityl alcohol, stearyl alcohol, behenyl alco-hol, hexadecyl alcohol, oleyl alcohol, isostearyl alcohol, hexyl dode-canol, octyl dodecanol, cetostearyl alcohol, 2-decyl tetradecynol, cho-lesterol, phytosterol, POE cholesterol ether and monostearyl glycerin ether.

Ester oils suitable as the component (C) include diisobutyl adipate, 2-hexyldecyl adipate, di-2-heptylundecyl adipate, N-alkylglycol monoisostearates, isocetyl isostearate, trimethylolpropane triisostearate, ethyleneglycol di-2-ethylhexanoate, cetyl 2-ethylhexanoate, trimethylolpropane tri-2-ethylhexanoate, pentaerithritol tetra-2-ethylhexanoate, cetyl octanoate, octyl dodecyl gum ester, oleyl oleate, octyldodecyl oleate, decyl oleate, neopentylglycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerithritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate and diisostearyl malate.

Glyceride oils suitable as the component (C) include acetoglyceride, glycerin triisooctanoate, glycerin triisostearate, glycerin triisopalmitate, glycerin tri-2-ethylhexanoate, glycerin monostearate, glycerin di-2-heptylundecanoate and glycerin trimyristate.

Silicone oils suitable as the component (C) include dimethylpolysiloxanes, methylphenylpolysiloxanes, methylhydrogenpolysiloxanes, octamethyl cyclotetrasiloxane, decamethyl cyclopentasiloxane, doecamethyl cyclohexasiloxane, tetramethyl cyclotetrasiloxane, higher alcohol-modified silicones such as stearyloxy silicones and higher fatty acid-modified silicones. Fluorocarbon oils suitable as the component (C) include perfluoro polyethers, perfluoro Decalin and perfluorooctane.

The above named oily materials can be used as the component (C) either singly or as a combination of two kinds or more according to need.

Besides the above described essential ingredients, i.e. components (A), (B) and (C), a great variety of additives can be compounded in the inventive makeup cosmetic composition depending on the particular types of the makeup composition as selected from those conventionally formulated in makeup cosmetic compositions including water, alcohols, water-soluble polymeric compounds, film-forming agents, surface active agents, oil-soluble gelation agents, resins, ultraviolet absorbers, moisturizing agents, antiseptic agents, antimicrobial agents, perfumes, salts, antioxidants, pH adjusting agents, chelating agents, refrigerant agents, anti-inflammatory agents, skin-beautifying agents, vitamins, amino acids, nucleic acids, hormones and clathrate compounds either singly or as a combination of two kinds or more.

The above mentioned alcohols include lower alcohols such as methyl, ethyl and isopropyl alcohols and sugar alcohols such as sorbitol and maltose.

The water-soluble polymeric compounds mentioned above include vegetable-origin polymeric compounds such as gum arabic, tragacanth gum, galactan gum, carob gum, guar gum, karaya gum, carageenan, pectin, agar, quince gum, starches of rice, Indian corn, potatoes, wheat and others, algae colloids and locust bean gum, microbeorigin polymeric compounds such as xanthan gum, dextran, succinoglucan and pullulan, animal-origin polymeric compounds such as collagen, casein, albumin and gelatin, starch-based polymeric compounds such as carboxymethyl starch and methyl hydroxypropyl starch, cellulose-based polymeric compounds such as methyl cellulose, ethyl cellulose, methyl hydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose and cellulose powder, alginic acid-based polymeric compounds such as sodium alginate and alginic acid ester of propyleneglycol, vinylic polymeric compounds such as polyvinyl methyl ether and carboxy divinyl polymer, polyoxyethylene-based polymeric compounds, polyoxyethylene-polyoxypropylene copolymeric compounds, acrylic polymeric compounds such as sodium polyacrylate, polyethyl acrylate and polyacrylamide, cation polymers, and so on. Certain film-forming agents such as polyvinyl alcohol and polyvinyl pyrrolidone are also included in this category of water-soluble polymeric compounds.

The surface active agents mentioned above can be any of anionic, cationic, non-ionic and amphoteric ones. Examples of the anionic surface active agents include fatty acid soaps such as sodium stearate and triethanolamine palmitate, alkyl ether carboxylic acids and salts thereof, carboxylates as a condensation product of an amino acid and a fatty acid, alkyl sulfonates, alkene sulfonates, sulfonates of a fatty acid ester, sulfonates of a fatty acid amide, sulfonates of a condensation product of an alkyl sulfonate and formaldehyde, sulfates, e.g., alkyl sulfates, secondary higher alcohol sulfates, alkyl ether sulfates, aryl ether sulfates, sulfates of a fatty acid ester, sulfates of a fatty acid alkylolamide and sulfate of Turkey red oil, alkyl phosphates, alkyl ether phosphates, amidophosphates, alkyl aryl ether phosphates and N-acylamino acid-based surface active agents. Examples of the cationic surface active agents include salts of amines such as salts of an alkylamine, polyamine and fatty acid derivatives of a polyamine and aminoalcohol, alkyl quaternary ammonium salts, aryl quaternary ammonium salts, pyridinium salts and imidazolium salts.

Examples of the non-ionic surface active agents include sorbitan fatty acid esters, glycerin fatty acid esters, polyglycerin fatty acid esters, propyleneglycol fatty acid esters, polyethyleneglycol fatty acid esters, fatty acid esters of sucrose, polyoxyethylene alkyl ethers, polyoxypropylene alkyl ethers, polyoxyethylene alkylphenyl ethers, polyoxyethylene fatty acid esters, polyoxyethylene-sorbitan fatty acid esters, polyoxyethylene-sorbitol fatty acid esters, polyoxyethylene-glycerin fatty acid esters, polyoxyethylene-propyleneglycol fatty acid esters, polyoxyethylene castor oils, polyoxyethylene hydrogenated castor oils, polyoxyethylene phytostanol ethers, polyoxyethylene phytosterol ethers, polyoxyethylene cholestanol ethers, polyoxyethylehe cholesteryl ethers, polyoxyalkylene-modified organopolysiloxanes, polyoxyalkylene-alkyl-comodified organopolysiloxanes, alkanolamides, sugar ethers and sugar amides. Examples of the amphoteric surface active agents include betaines, aminocarboxylates and imidazoline derivatives.

The oil-soluble gelation agent mentioned above is exemplified by amino acid derivatives such as N-lauroyl-L-glutamic acid and α, γ-di-n-butyl amine, dextrin fatty acid esters such as dextrin palmitate, dextrin stearate and dextrin 2-ethylhexanoate palmitate, sucrose fatty acid esters such as sucrose palmitate and sucrose stearate, benzylidene derivatives of sorbitol such as monobenzylidene sorbitol and dibenzylidene sorbitol and so on.

The above mentioned ultraviolet absorber includes benzoic acid-based ultraviolet anbsorbers such as p-amino benzoic acid, anthranilic acid-based ultraviolet absorbers such as methyl anthranilate, salicylic acid-based ultraviolet absorbers such as methyl salicylate, cinnamic acid-based ultraviolet absorbers such as octyl 4-methoxycinnamate, benzophenone-based ultraviolet absorbers such as 2,4-dihydroxy benzophenone, urocanic acid-based ultraviolet absorbers such as ethyl urocanate, and so on.

The moisturizing agent is exemplified by sorbitol, xylitol, propyleneglycol, dipropyleneglycol, 1,3-butyleneglycol, glycerin, diglycerin, polyethyleneglycol, hyaluronic acid, condroitin sulfate and pyrrolidone carboxylates.

The antiseptic agent is exemplified by alkyl paraoxybenzoates, benzoic acid, sodium benzoate, sorbic acid, potassium sorbate and phenoxyethanol. The antimicrobial agent is exemplified by benzoic acid, salicylic acid, phenol, sorbic acid, paraoxybenzoates, p-chloro m-cresol, hexachlorophene, benzalkonium chloride, trichloro carbanilide and phenoxy ethanol.

The antioxidant is exemplified by tocopherol, butyl hydroxyanisole (BHA) and dibutyl hydroxytoluene (BHT). The pH controlling agent is exemplified by lactic acid, citric acid, glycolic acid, succinic acid, tartaric acid, dl-malonic acid, potassium carbonate, sodium hydrogencarbonate and ammonium hydrogencarbonate. The chelating agent is exemplified by alanine, sodium edetate, sodium polyphosphate, sodium metaphosphate and phosphoric acid. The refrigerant agent is exemplified by L-menthol and camphor. The anti-inflammatory agent is exemplified by alantoin, glycyrrhetinic acid, tranexamic acid and azulene.

The skin beautifying agents include skin-whitening agents such as placenta extract, arbutin, glutathione and saxifrage extract, cell-invigorating agents such as royal jelly, cholesterol derivatives and juvenile bovine blood extract, rough skin improvers, blood circulation promoters such as benzyl nicotinate, 2-butoxyethyl nicotinate, capsaicine, zingerone, cantharis tincture, ichthammol, caffein, tannic acid, α-borneol, tocopherol nicotinate, inocitol hexanicotinate, cyclandelate, tolazoline, acetyl choline, cepharanthine and γ-orizanol, skin astringents such as tannic acid, and antiseborrheic agents such as sulfur and thianthol.

The vitamin is exemplified by vitamin A compounds such as vitamin A oil, retinol, retinol acetate and retinol palmitate, vitamin B₂ compounds such as riboflavin, riboflavin butyrate and flavinadenine nucleotide. vitamin B₆ compounds such as pyridoxine hydrochloride and pyridoxine dioctanoate, vitamin C compounds such as L-ascorbic acid, L-ascorbic acid dipalmitate, sodium L-ascorbic acid disulfate and dl-α-tocopherol-L-ascorbic acid phosphoric acid diester dipotassium, pantothenic acid compounds such as calcium pantothenate, D-pantothenyl alcohol, pantothenyl ethyl ether and acetylpantothenyl ethyl ether, vitamin D compounds such as ergocalciferol and cholecalciferol, nicotinic acid compounds such as nicotinic acid, benzyl nicotinate and nicotinic amide, vitamin E compounds such as dl-α-topopherol, dl-α-tocopherol acetate, dl-α-tocopherol nicotinate and dl-α-tocopherol succinate, vitamin P, biotin and so on.

The amino acid is exemplified by arginine, aspartic acid, cystine, cysteine, methionine, serine, leucine and tryptophane. The nucleic acid is exemplified by deoxy ribonucleic acid. The hormone is exemplified by estradiol and ethenyl estradiol.

The makeup cosmetic composition of the present invention can be prepared in a variety of forms including powders, powder compacts, milky lotions, liquid suspensions, sticks, mousses and sprays, which can be water-based, milky or oil-based. Particular preparation forms include face powders, foundations, cheek rouges, eye shadows, lip sticks, eye liners, mascaras, eyebrow pencils and manicures

In the following, the makeup cosmetic composition of the present invention is described in more detail by way of examples, which, however, never limit the scope of the invention in any way, as preceded by a description of the preparation procedure of the composite silicone particles as the component (A).

The foundations prepared in Example 1 and Comparative Examples 1 and 2 were each subjected to organoleptic evaluation tests of use for the nine items (a) to (i) shown below by 50 female expert panel members. Namely, each of the panel members was requested to report her evaluation of the tested foundation composition for (a) take-up of the composition on the puff or sponge, (b) stability against caking, (c) non-sticky and dry feeling in makeup finishing, (d) spreadability of the composition over the skin, (e) smoothness of the skin after makeup, (f) fitness of makeup finish to the skin, (g) absence of stickiness on the skin after makeup finish, (h) aesthetic view of cosmetic finish and (i) sustainability of makeup.

The results of their evaluation for each item were reported in five ratings giving 1 to 5 points according to the following criteria.
5: excellent
4: good
3: fair
2: somewhat poor
1: unacceptable

The values of points given by 50 panel members were averaged for each testing item and the average values for the respective testing items were rated in four ratings of A, B, C and D depending on the average points as follows.
A: 4.5 points or higher
B: 3.5 points or higher but lower than 4.5 points
C: 2.5 points or higher but lower than 3.5 points
D: lower than 2.5 points

### Preparation 1.

Into a glass beaker of 1 liter capacity were taken, under agitation with a homomixer rotating at 2000 rpm, 500 g of a dimethyl polysiloxane terminated at each molecular chain end with a vinyl group and having a viscosity of 600 centistokes at 25 °C as expressed by the structural formula

Vi-SiMe₂-O-(-SiMe₂-O-)₁₈₀-SiMe₂-Vi,

In which Me is a methyl group and Vi is a vinyl group, and 20 g of a methyl hydrogen polysiloxane having a viscosity of 30 centistokes at 25 °C as expressed by the structural formula

Me₃Si-O-(-SiHMe-O-)₁₀-(-SiMe₂-O-)₃₀-SiMe₃,

In which Me is a methyl group, to give a siloxane mixture, which was admixed with 1 g of a polyoxyethylene (9 moles ethylene oxide addition) octylphenyl ether as a surface active agent and 150 g of water and agitated at 6000 rpm to give an oil-in-water emulsion. Thereafter, the emulsion was further admixed with 329 g of water and agitated at 2000 rpm to give a diluted oil-in-water emulsion.

The thus prepared emulsion was transferred into a glass flask of 1 liter capacity equipped with an anchor-blade stirrer and admixed with a mixture of 1 g of a toluene solution of a chloroplatinic acid-olefin complex containing 0.05% of platinum and 1 g of the same surface active agent as used above and kept for 12 hours at room temperature under agitation for effecting the hydrosilation reaction to give an aqueous dispersion of spherical cured silicone rubber particles having an average particle dimeter of 15 µm as determined by a Coulter Counter (manufactured by Coulter Electronics Co.).

A 580 g portion of the above obtained aqueous suspension of spherical cured silicone rubber particles was taken in a glass flask of 3 liters capacity together with 2290 g of water and 60 g of a 28% ammonia water to give a reaction mixture into which, under agitation with an anchor-blade stirrer rotating at 200 rpm, 65 g of methyl trimethoxy silane were added dropwise over a period of 20 minutes at 10 °C. After completion of the dropwise addition of the silane compound, agitation of the reaction mixture was further continued first at 5 to 15 °C for 4 hours and then at 55 to 60 °C for 1 hour to complete the hydrolysis-condensation reaction of the silane compound depositing a cladding layer of polymethylsilsesquioxane on the cured rubber particles as the core. The reaction mixture was filtered through a pressurizable filter to give a wet cake of particles containing about 30% of water which was dried in a hot-air circulation oven at 105°C followed by disintegration of the dried cake in a jet mill to give fine spherical composite silicone particles, referred to as the composite powder 1 hereinafter, each consisting of a core of the spherical cured silicone rubber particle and a cladding layer of a polymethylsilsesquioxane resin derived from methyl trimethoxy silane.

The composite particles as dispersed in water were subjected to the measurement of the average particle diameter by using a Coulter Counter to obtain a value of 15 µm. A gravimetric analysis of the particles indicated that the composite particles consisted of 100 parts by weight of the core particles and 10 parts by weight of the cladding layers.

### Preparation 2.

The procedure for the preparation of composite silicone particles, referred to as the composite powder 2 hereinafter, was substantially the same as in Preparation 1 described above excepting for the replacement of 65 g of methyl trimethoxy silane with a mixture of 55 g of methyl trimethoxy silane and 10 g of 3-glycidyloxypropyl trimethoxy silane. The composite silicone particles had a spherical particle configuration and an average particle diameter of 15 µm as determined in the same manner as in Preparation 1. The amount of the cladding layers of the polyorganosilsesquioxane resin was 11 parts by weight per 100 parts by weight of the core particles of the cured silicone rubber.

### Preparation 3.

The procedure for the preparation of spherical composite silicone particles, referred to as the composite powder 3 hereinafter, was substantially the same as in Preparation 1 described above except that the aqueous suspension of spherical cured silicone rubber particles was prepared by increasing the amount of the polyoxyethylene octylphenyl ether as the surface active agent in the preparation of the oil-in-water aqueous emulsion of mixed organopolysiloxanes from 1 g in Preparation 1 to 5 g so that the spherical cured silicone rubber particles in the aqueous suspension had an average particle diameter of 3 µm as determined by using a Coulter Counter. The amount of the cladding layers of the polymethylsilsesquioxane resin was 10 parts by weight per 100 parts by weight of the core particles of the cured silicone rubber.

### Example 1.

A foundation as a makeup cosmetic composition was prepared according to the following formulation of 15 ingredients given in parts by weight. The results of the organoleptic evaluation tests are shown in Table 1.

| | | |
|---|---|---|
| (1) | Titanium dioxide | 12.0 parts by weight |
| (2) | Zinc oxide | 9.5 |
| (3) | Kaolin | 35.0 |
| (4) | Talc | 20.0 |
| (5) | Red iron oxide | 0.8 |
| (6) | Yellow iron oxide | 2.5 |
| (7) | Black iron oxide | 0.2 |
| (8) | Composite powder 1 | 7.0 |
| (9) | Liquid paraffin | 4.0 |
| (10) | Octamethyl cyclotetrasiloxane | 5.0 |
| (11) | Dimethylpolysiloxane | 5.0 |
| (12) | Isopropyl palmitate | 3.0 |
| (13) | Glycerin | 3.0 |
| (14) | Antiseptic agent | q.s. |
| (15) | Perfume | q.s. |

The ingredients (1) to (8) were mixed together to give a uniform mixture which was added to a mixture of the ingredients (9) to (14) separately prepared. The thus prepared mixture of the ingredients (1) to (14) was finally admixed with the ingredient (15). The thus obtained mixture was compression-compacted in a metal dish into a powder compact.

### Comparative Example 1.

A comparative foundation was prepared in just the same formulation as in Example 1 excepting for the replacement of the composite powder 1 with the same amount of a polymethylsilsesquioxane powder which was a commercial product consisting of spherical particles having an average particle diameter of 12 µm (Tospearl 3120, a product by Toshiba Silicone Co.).

The results of the organoleptic evaluation tests of this comparative foundation are shown in Table 1.

### Comparative Example 2.

Another comparative foundation was prepared in just the same formulation as in Example 1 excepting for the replacement of the composite powder 1 with the same amount of a cured silicone rubber powder which was a commercial product consisting of spherical particles having an average particle diameter of 10 µm (Torayfil E501, a product by Toray Dow Corning Co.).

The results of the organoleptic evaluation tests of this comparative foundation are shown in Table 1.

**Table 1**

| Testing Item | Example 1 | Comparative Example 1 | Comparative Example 2 |
|---|---|---|---|
| (a) | A | B | D |
| (b) | A | B | D |
| (c) | A | A | A |
| (d) | A | A | A |
| (e) | A | D | B |
| (f) | A | C | C |
| (g) | A | B | B |
| (h) | A | C | C |
| (i) | A | C | C |
| overall | A | D | D |

### Example 2.

A pressed-powder foundation was prepared from 12 ingredients according to the following formulation.

| | | |
|---|---|---|
| (1) | Titanium dioxide | 5.0 parts by weight |
| (2) | Sericite | 10.0 |
| (3) | Kaolin | 5.0 |
| (4) | Talc | 52.0 |
| (5) | Zinc myristate | 5.0 |
| (6) | Colored pigments | 3.0 |
| (7) | Composite powder 2 | 5.0 |
| (8) | Spherical porous silica particles | 10.0 |
| (9) | Squalane | 3.0 |
| (10) | Gluceryl trioctanoate | 2.0 |
| (11) | Antiseptic agent | q.s. |
| (12) | Perfume | q.s. |

In the first place, the ingredients (1) to (8) were blended together to give a blend and then the ingredients (9) to (11) were added thereto followed by final addition of the ingredient (12) into a uniform blend, which was compacted in a metal dish by compression to give a caked foundation.

The thus prepared pressed-powder foundation was subjected to the evaluation tests by panel members who reported that the foundation was excellent in respects of smooth and very dry touch feeling, good spreadability over the skin, fitness on the skin, beauty of cosmetic finishing and good sustainability of the cosmetic finish.

### Example 3.

An eye shadow stick was prepared from 13 ingredients according to the following formulation.

| | | |
|---|---|---|
| (1) | Gypsum | 35.0 parts by weight |
| (2) | Mica flakes | 10.0 |
| (3) | Talc | 17.5 |
| (4) | Pigments | 10.0 |
| (5) | Nylon Powder | 15.0 |
| (6) | Composite powder 3 | 5.0 |
| (7) | Squalane | 3.0 |
| (8) | Polyoxyethylene sorbitan monooleate | 0.5 |
| (9) | Glycerin | 4.0 |
| (10) | Antiseptic agent | q.s. |
| (11) | Perfume | q.s. |
| (12) | Purified water | q.s. |
| (13) | Ethanol (50%) | q.s. |

The ingredients (1) to (6) and ingredients (7) to (13) were respectively blended to give two blends which were then mixed together to give an eye shadow composition which was put into a capsule for an eye shadow stick and kept standing therein to effect hardening followed by drying.

The thus prepared eye shadow stick was subjected to the evaluation tests by panel members who reported that the eye shadow composition was excellent in respects of smooth and very dry touch feeling, good spreadability over the skin, fitness on the skin, beauty of cosmetic finish and good sustainability of cosmetic finish. Further, the eye shadow had good use feasibility in respect of easy gradation-making without powderiness.

### Example 4.

A cheek rouge composition was prepared from 11 ingredients according to the following formulation.

| | | |
|---|---|---|
| (1) | Titanium | 3.0 parts by weight |
| (2) | Mica flakes | 11.0 |
| (3) | Colored pigments | 1.0 |
| (4) | Composite Powder 1 | 10.0 |
| (5) | Non-porous spherical silica particles | 20.0 |
| (6) | Ceresin | 4.0 |
| (7) | Candelilla wax | 1.0 |
| (8) | Squalane | 35.0 |
| (9) | Dimethylpolysiloxane | 15.0 |
| (10) | Antiseptic agent | q.s. |
| (11) | Perfume | q.s. |

A mixture of the ingredients (6) to (10) in a molten state under heating was admixed with a mixture of the ingredients (1) to (5) followed by final addition of the ingredient (11) to give a uniform blend which was poured into a case and cooled therein.

The thus prepared cheek rouge composition was subjected to the evaluation tests by panel members who reported that the cheek rouge was excellent in respects of smooth and very dry touch feeling, good spreadability over the skin, fitness on the skin, beauty of cosmetic finish and good sustainability of cosmetic finish.

## Claims

1. A makeup cosmetic composition which comprises, as a uniform blend:
(A) from 0.01 to 30% by weight of a powder of composite silicone particles, esch particle consisting of a core of a cured silicone rubber and a cladding layer of a polyorganosilsesquioxane resin formed on the core;
(B) a non-silicone powder; and
(C) an oiling agent.

2. The makeup cosmetic composition as claimed in claim 1 in which the content of the component (A) is in the range from 0.05 to 10% by weight.

3. The makeup cosmetic composition as claimed in claim 1 in which the polyorganosilsesquioxane is a polymethylsilsesquioxane.

4. The makeup cosmetic composition as claimed in claim 1 in which the composite silicone particles have a spherical particle configuration.

5. The makeup cosmetic composition as claimed in claim 4 in which the composite silicone particles have an average particle diameter in the range from 0.1 to 100 µm.

6. The makeup cosmetic composition as claimed in claim 1 in which the amount of the cladding layers of a polyorganosilsesquioxane resin on the core is in the range from 1 to 20% by weight based on the amount of the composite silicone particles.

7. The makeup cosmetic composition as claimed in claim 1 in which the cured silicone rubber is a product of a hydrosilation reaction between a vinyl group-containing organopolysiloxane and an organohydrogenpolysiloxane mixed together in the form of an oil-in-water emulsion in an aqueous medium.

8. The makeup cosmetic composition as claimed in claim 1 in which the polyorganosilsesquioxane resin is a hydrolysis-condensation product of an organotrialkoxy silane compound in an aqueous medium in the presence of the core particles of a cured silicone rubber.

9. The makeup cosmetic composition as claimed in claim 8 in which the organotrialkoxy silane compound is methyl trimethoxy silane.
